# FASCICULE DE BREVET EUROPEEN

(11) **EP 3 452 206 B1**
(45) Date de publication et mention de la délivrance du brevet: **06.11.2019**
(21) Numéro de dépôt: 17721773.4
(22) Date de dépôt: 11.04.2017
(51) Int. Cl.: B01D 53/68, B01D 53/70, C01B 7/07, C07C 17/389

(54) **PROCEDE ET INSTALLATION DE PURIFICATION D'ACIDE CHLORHYDRIQUE**
SALZSÄUREREINIGUNGSVERFAHREN UND -ANLAGE
HYDROCHLORIC ACID PURIFICATION PROCESS AND PLANT

(30) Priorité: 03.05.2016 FR 1654010
(43) Date de publication de la demande: 13.03.2019
(73) Titulaire: Arkema France, 92700 Colombes (FR)
(72) Inventeur: LAUNAY, Marianne, 69360 Serezin du Rhone (FR); EMERY, Irène, 69340 FRANCHEVILLE (FR); BIDET, David Jean-Léopold, 69600 Oullins (FR)
(74) Mandataire: Albani, Dalila
(86) Numéro de dépôt international: PCT/FR2017/050864
(87) Numéro de publication internationale: WO 2017/191388

(56) Documents cités:
- WO-A1-2015/079137
- FR-A- 1 129 260
- FR-A- 1 507 252
- US-A- 3 387 430

## Description

### OBJET DE L'INVENTION

La présente invention concerne un procédé de purification d'acide chlorhydrique, ainsi qu'une installation adaptée à la mise en œuvre de ce procédé. L'invention peut être en particulier utilisée dans le cadre du traitement des sous-produits ou des effluents issus de la synthèse d'un composé fluoré à partir d'un composé ogano-halogéné.

### ARRIERE-PLAN DE L'INVENTION

Il est connu de produire des composés fluorés tels que les hydrofluorocarbures par fluoration de composés chlorés tels que les hydrochlorocarbures. Cette fluoration est généralement une fluoration catalytique utilisant l'acide fluorhydrique (HF) comme agent fluorant. Au cours de ce type de réaction, de l'acide chlorhydrique (HCl) est co-produit. En pratique, il n'est par ailleurs pas toujours possible ou souhaitable de faire réagir la totalité du HF mis en jeu. Ce dernier se retrouve alors sous forme d'impureté associée à l'acide chlorhydrique. D'autres réactions de synthèse de composés fluorés conduisent également à la co-production de HCl pollué par une faible quantité d'HF. Tel est notamment le cas de la fabrication du fluorure de vinylidène par pyrolyse du 1-chloro-1,1-difluoroéthane.

Dans la mesure où l'acide chlorhydrique ne peut être rejeté dans l'environnement, des techniques ont été proposées pour le valoriser. Il est ainsi connu de séparer le HCl des autre gaz produits (y compris les composés fluorés d'intérêt) par absorption adiabatique pour générer une solution d'HCl de type commercial et récupérer en tête les gaz bruts à traiter. Dans le cas où l'acide chlorhydrique est co-produit dans un procédé de pyrolyse, il a toutefois été observé que la pyrolyse pouvait entraîner la formation de goudrons susceptibles d'encrasser la colonne adiabatique. En outre, la solution de HCl ainsi obtenue est polluée non seulement par de faibles quantités d'acide fluorhydrique (HF), mais aussi par des résidus organiques de pyrolyse et des composés organo-halogénés qui constituent des sous-produits de la pyrolyse. Cette solution de HCl n'est donc pas suffisamment pure pour la plupart des applications.

Des procédés ont été proposés pour améliorer la pureté d'une solution de HCl polluée par du HF et éventuellement des composés organiques halogénés.

Ainsi, le document FR 1507252 décrit un procédé visant à séparer l'acide fluorhydrique mélangé à HCl, consistant à faire passer ce mélange gazeux dans un dispositif de lavage continu à contre-courant comprenant une colonne à plateaux, le lavage étant effectué à l'aide d'une solution aqueuse concentrée d'acide chlorhydrique à basse température, qui est capable d'absorber l'acide fluorhydrique. On obtient en sortie de cette colonne une solution aqueuse d'acide chlorhydrique et d'acide fluorhydrique, ainsi que de l'acide chlorhydrique gazeux, appauvri en HF par rapport à l'acide chlorhydrique de départ. Pour obtenir de l'acide chlorhydrique quasiment exempt de HF, il est toutefois nécessaire de faire passer le mélange de gaz brut sur du charbon actif en présence d'eau, en amont de l'étape de lavage acide. On comprend que ce procédé n'est donc pas applicable à un mélange de gaz brut renfermant un gaz fluoré d'intérêt, qui se trouverait alors piégé dans le charbon actif.

Il a par ailleurs été suggéré dans le document WO 2015/079137 que l'acide chlorhydrique co-produit dans un procédé de fluoration catalytique pouvait être purifié suivant un procédé impliquant une étape d'hydrolyse catalytique des composés fluoro-oxygénés contenus dans le flux gazeux brut, suivie d'une étape de lavage par une solution acide puis d'une étape d'adsorption des gaz sur un lit de charbon actif, et enfin d'une étape d'adsorption adiabatique permettant d'obtenir une solution aqueuse d'acide chlorhydrique. Cette solution peut éventuellement être ultérieurement purifiée sur gel de silice. Ce procédé intègre nécessairement une étape préliminaire de distillation des gaz bruts afin d'éviter que le produit fluoré d'intérêt ne se trouve piégé dans le lit de charbon actif mis en œuvre dans l'étape d'hydrolyse catalytique. Il serait donc souhaitable de disposer d'un procédé qui ne soit pas plus complexe que celui décrit dans la demande WO 2015/079137, mais qui permette de traiter directement le flux gazeux brut issu de la réaction catalytique, sans qu'il ne soit nécessaire de le soumettre à une distillation préalable.

### RESUME DE L'INVENTION

Il subsiste donc le besoin de proposer un procédé amélioré de purification de l'acide chlorhydrique contenu dans un flux gazeux brut, en mélange avec de l'acide fluorhydrique, un composé fluoré d'intérêt et des composés organiques halogénés, lequel procédé permette de réduire à la fois le taux d'acide fluorhydrique et de composés organiques et avantageusement d'atteindre une concentration finale élevée en acide chlorhydrique.

Les inventeurs ont démontré que ce besoin pouvait être satisfait en sélectionnant une succession d'étapes de traitement particulières, agencées dans un ordre déterminé.

L'invention a ainsi pour premier objet un procédé de traitement d'un flux gazeux contenant de l'acide chlorhydrique, de l'acide fluorhydrique, un composé fluoré d'intérêt et des composés organiques halogénés, dans lequel le flux gazeux est successivement soumis à :
(a) une étape de lavage par une solution acide pour obtenir un flux gazeux lavé ;
(b) une étape d'absorption adiabatique dans une solution aqueuse de l'acide chlorhydrique contenu dans ledit flux gazeux lavé, permettant de collecter une solution d'acide chlorhydrique et un flux gazeux renfermant ledit composé fluoré d'intérêt ;
(c) une étape d'adsorption sur charbon actif des impuretés organiques présentes dans ladite solution d'acide chlorhydrique, pour obtenir une solution d'acide chlorhydrique purifiée ; et
(d) une étape de mise en contact de ladite solution d'acide chlorhydrique purifiée avec un gel de silice.

Un second objet de l'invention porte sur un procédé de préparation d'un composé fluoré comprenant :
- la fluoration catalytique d'un composé organochloré avec de l'acide fluorhydrique ou la pyrolyse, catalytique ou non, d'un composé organofluoré comprenant au moins un atome de chlore,
- la collecte d'un flux gazeux brut contenant de l'acide chlorhydrique, de l'acide fluorhydrique, ledit composé fluoré d'intérêt et des composés organiques halogénés ; et
- le traitement dudit flux gazeux brut suivant le procédé décrit ci-dessus.

L'invention a pour troisième objet une installation de traitement d'un flux gazeux brut contenant de l'acide chlorhydrique, de l'acide fluorhydrique, un composé fluoré d'intérêt et des composés organiques halogénés, comprenant :
- une unité de lavage comprenant une colonne de lavage alimentée, d'une part, par une conduite d'amenée dudit flux gazeux brut et, d'autre part, par une conduite d'amenée de solution acide ;
- une unité d'absorption adiabatique comprenant une colonne alimentée, d'une part, par une conduite de collecte de flux lavé issue de l'unité de lavage et d'autre part par une conduite d'amenée de solution aqueuse ;
- une conduite de collecte du composé fluoré d'intérêt sous forme gazeuse, en sortie de colonne de l'unité d'adsorption adiabatique,
- une unité d'adsorption d'impuretés organiques, comprenant un lit de charbon actif alimenté par une conduite de collecte de solution d'acide chlorhydrique issue de l'unité d'adsorption adiabatique ;
- une unité d'adsorption complémentaire reliée à une conduite de collecte de solution purifiée issue de l'unité d'adsorption d'impuretés, et comprenant une colonne renfermant un gel de silice.

Dans une forme d'exécution préférée de l'invention, l'installation comprend en outre, au sein de l'unité de lavage, une unité de séparation de composés lourds disposée en amont de la colonne de lavage, ladite unité de séparation de composés lourds étant alimentée par une conduite d'amenée du flux brut et reliée à la colonne de lavage par une conduite de collecte de flux gazeux dégoudronné.

Le procédé et l'installation selon l'invention permettent de produire, à l'issue de l'étape (d), une solution aqueuse d'acide chlorhydrique renfermant au moins 30% en poids d'acide chlorhydrique, moins de 10 ppm, voire moins de 5 ppm, mieux, moins de 1 ppm, d'acide fluorhydrique et moins de 150 ppm, voire moins de 100 ppm, mieux, moins de 50 ppm, de composés organiques halogénés.

### FIGURES

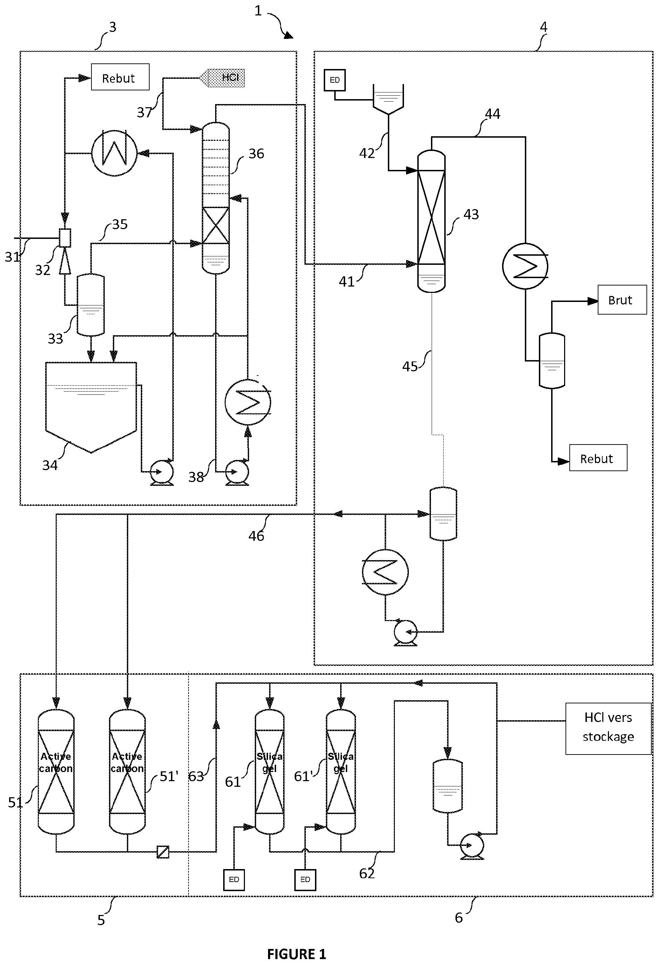

La figure 1 représente de manière schématique un mode de réalisation d'une installation selon l'invention.

### DESCRIPTION DETAILLEE

L'invention est maintenant décrite plus en détail et de façon non limitative dans la description qui suit.

L'invention s'applique en particulier au traitement d'un flux gazeux issu d'une réaction de synthèse d'un composé fluoré, notamment par pyrolyse, catalytique ou non, d'un composé organofluoré comprenant au moins un atome de chlore, ou par fluoration catalytique d'un composé organochloré avec de l'acide fluorhydrique, de préférence par pyrolyse catalytique d'un composé organofluoré comprenant au moins un atome de chlore. Le composé fluoré issu de ces réactions est désigné dans cette description par "composé fluoré d'intérêt". Il se distingue des composés chlorés et/ou fluorés obtenus à titre de co-produits à l'issue de ces réactions, ainsi que des réactifs utilisés. Ce composé fluoré d'intérêt peut être en particulier le fluorure de vinylidène (VF2).

Par composé organochloré, on entend un composé organique comprenant un ou plusieurs atomes de chlore, et par composé organofluoré, on entend un composé organique comprenant un ou plusieurs atomes de fluor. Il est entendu que le composé organochloré peut comprendre un ou plusieurs atomes de fluor, de même que le composé organofluoré renferme au moins un atome de chlore.

Le composé organochloré peut être un alcane ou un alcène ayant au moins un substituant Cl et éventuellement au moins un substituant choisi parmi F, I et Br (de préférence F). Le composé organofluoré peut être un alcane ou un alcène ayant au moins un substituant F et Cl et éventuellement au moins un substituant choisi parmi I et Br. Le composé organochloré et le composé organofluoré peuvent être linéaires ou ramifiés, de préférence linéaires.

L'invention trouve notamment à s'appliquer pour les réactions de fluoration suivantes :
- fluoration du perchloroéthylène (PER) en pentafluoroéthane (HFC-125) ;
- fluoration du 1,1,1,2,3-pentachloropropane (HCC-240db) en 2-chloro-3,3,3-trifluoropropène (HCFO-1233xf) ;
- fluoration du 1,1,1,2,3-pentachloropropane (HCC-240db) en 2,3,3,3-tétrafluoropropène (HFO-1234yf) ;
- fluoration du 1,1,1,3,3-pentachloropropane (HCC-240fa) en 1,3,3,3-tétrafluoropropène (HFO-1234ze) ;
- fluoration du 1,1,1,3,3-pentachloropropane (HCC-240fa) en 1 - chloro-3,3,3-trifluoropropène (HCFO-1233zd) ;
- fluoration du 2-chloro-3,3,3-trifluoropropène (HCFO-1233xf) en 2,3,3,3-tétrafluoropropène (HFO-1234yf) ;
- fluoration du 1,1,2,2,3-pentachloropropane (HCC-240aa) en 2,3,3,3-tétrafluoropropène (HFO-1234yf) ;
- fluoration du 1,1,2,2,3-pentachloropropane (HCC-240aa) en 2-chloro-3,3,3-trifluoropropène (HCFO-1233xf) ;
- fluoration du 2,3-dichloro-1,1,1-trifluoropropane (HCFC-243db) en 2,3,3,3-tétrafluoropropène (HFO-1234yf) ;
- fluoration du 2,3-dichloro-1,1,1-trifluoropropane (HCFC-243db) en 1-chloro-3,3,3-trifluoropropène (HCFO-1233zd) ;
- fluoration du 2,3-dichloro-1,1,1-trifluoropropane (HCFC-243db) en 1,3,3,3-tétrafluoropropène (HFO-1234ze) ;
- fluoration du 2,3-dichloro-1,1,1-trifluoropropane (HCFC-243db) en 2-chloro-3,3,3-trifluoropropène (HCFO-1233xf) ;
   fluoration du 1 ,1 ,2,3-tétrachloropropène (HCO-1230xa) en 2,3,3,3- tétrafluoropropène (HFO-1234yf) ;
- fluoration du 1,1,2,3-tétrachloropropène (HCO-1230xa) en 2-chloro-3,3,3-trifluoropropène (HCFO-1233xf) ;
- fluoration du 2,3,3,3-tetrachloropropène (HCO-1230xf) en 2,3,3,3- tétrafluoropropène (HFO-1234yf) ;
- fluoration du 2,3,3,3-tétrachloropropène (HCO-1230xf) en 2-chloro-3,3,3-trifluoropropène (HCFO-1233xf) ;
- fluoration du 1,1,3,3-tétrachloropropène (HCO-1230za) en 1- chloro-3,3,3-trifluoropropène (HCFO-1233zd) ;
- fluoration du 1,1,3,3-tétrachloropropène (HCO-1230za) en 1,3,3,3-tétrafluoropropène (HFO-1234ze) ;
- fluoration du 1,3,3,3-tétrachloropropène (HCO-1230zd) en 1- chloro-3,3,3-trifluoropropène (HCFO-1233zd) ;
- fluoration du 1,3,3,3-tétrachloropropène (HCO-1230zd) en 1,3,3,3- tétrafluoropropène (HFO-1234ze) ;
- fluoration du 1-chloro-3,3,3-trifluoropropène (HCFO-1233zd) en 1,3,3,3-tétrafluoropropène (HFO-1234ze) ;
- fluoration du 1,1,2-trichloroéthane en 1-chloro,2,2-difluoroéthane (HCFC-142) ;
- fluoration du 1,2-dichloroéthylène en 1-chloro-2,2-difluoroéthane (HCFC-142).

La conversion du composé organochloré en composé fluoré d'intérêt peut être une conversion directe (avec une seule étape de réaction ou avec un seul ensemble de conditions réactionnelles) ou une conversion indirecte (avec deux ou plus de deux étapes de réaction ou en utilisant deux ou plus de deux ensembles de conditions réactionnelles). La conversion peut être totale ou partielle.

Afin d'éviter une désactivation rapide du catalyseur lors de la réaction, un agent d'oxydation (par exemple de l'oxygène ou du chlore) peut être ajouté. On peut par exemple utiliser un flux d'oxygène pur ou de chlore pur, ou un mélange oxygène / azote ou chlore / azote.

Le catalyseur utilisé peut être par exemple à base d'un métal comprenant un oxyde de métal de transition ou un halogénure ou un oxyhalogénure d'un tel métal. On peut citer par exemple FeCl₃, l'oxyfluorure de chrome, les oxydes de chrome (éventuellement soumis à des traitements de fluoration), les fluorures de chrome et leurs mélanges. D'autres catalyseurs possibles sont les catalyseurs supportés sur du carbone, les catalyseurs à base d'antimoine, les catalyseurs à base d'aluminium (par exemple AlF₃ et Al₂O₃, l'oxyfluorure d'aluminium et le fluorure d'aluminium). On peut utiliser en général un oxyfluorure de chrome, un fluorure ou un oxyfluorure d'aluminium, ou un catalyseur supporté ou non contenant un métal tel que Cr, Ni, Fe, Zn, Ti, V, Zr, Mo, Ge, Sn, Pb, Mg, Sb. On peut faire référence à cet égard au document WO 2007/079431 (en p.7, 1.1-5 et 28-32), au document EP 0939071 (paragraphe [0022]), au document WO 2008/054781 (en p.9 1.22-p.10 1.34), et au document WO 2008/040969 (revendication 1), auxquels il est fait expressément référence. Avant son utilisation, le catalyseur est de préférence soumis à une activation avec de l'air, de l'oxygène ou du chlore et/ou avec de l'HF. En variante, on peut utiliser le chlore comme catalyseur.

Dans une forme d'exécution préférée, le procédé selon l'invention est appliqué à un flux gazeux issu de la pyrolyse catalytique d'un composé organofluoré comprenant au moins un atome de chlore, plus particulièrement un flux gazeux issu de la déshydrochloration pyrolytique du 1-chloro-1,1-difluoroéthane en fluorure de vinylidène, selon la réaction suivante : 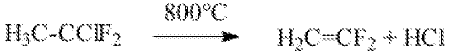 la réaction étant de préférence effectuée en présence de chlore comme catalyseur.

Le flux gazeux traité selon le procédé de l'invention, qui est mis en œuvre dans l'étape (a), peut être directement le flux gazeux brut issu de la réaction de synthèse du composé fluoré d'intérêt (tel que le fluorure de vinylidène), ou plus préférentiellement un flux gazeux résultant de la séparation des composés lourds contenus dans ce flux gazeux brut. Cette étape de séparation vise à séparer le flux gazeux à traiter selon l'invention d'une fraction organique lourde, contenant notamment des goudrons formés lors de la réaction. On préfère selon l'invention que le flux gazeux à traiter, issu de la réaction de pyrolyse catalytique ou de fluoration catalytique ou, le cas échéant, de l'étape de séparation de composés lourds, ne soit pas soumis préalablement à une étape de distillation.

Le flux gazeux à traiter selon le procédé de l'invention renferme de l'acide chlorhydrique, de l'acide fluorhydrique, le composé fluoré d'intérêt et des composés organiques halogénés, en particulier des composés organochlorés et/ou organofluorés. Ces composés organiques comprennent généralement le réactif organochloré ou organofluoré n'ayant pas réagi lors de la réaction de fluoration ou de pyrolyse catalytique, ainsi que les sous-produits de ces réactions. Ce flux gazeux comprend généralement moins de 30%, par exemple moins de 25% en poids, voire moins de 20% en poids, d'acide chlorhydrique. En particulier, lorsqu'il est issu de la réaction de pyrolyse catalytique illustrée ci-dessus, le flux gazeux à traiter selon l'invention contient par exemple du fluorure de vinylidène, de l'acide chlorhydrique, de l'acide fluorhydrique et des composés organiques halogénés, tels que des composés organochlorés et organofluorés.

Dans la première étape du procédé selon l'invention, ce flux gazeux est soumis à un lavage à l'aide d'une solution acide, qui permet de réduire la quantité d'acide fluorhydrique qu'il renferme. On pourra à cette fin utiliser le procédé de lavage acide décrit dans la demande FR 1 507 252, qui est incorporée ici par référence, ainsi que l'installation de lavage acide décrite ci-après.

Selon un mode de réalisation, la solution acide utilisée lors de l'étape de lavage est une solution d'acide chlorhydrique, à une concentration molaire pouvant aller par exemple de 5 à 60 %, notamment de 10 à 50 %, plus préférentiellement de 20 à 45 % et en particulier de 30 à 35 %. Le lavage par la solution acide est de préférence mis en œuvre à une température de 5 à 50°C, et plus particulièrement de 7 à 40°C et/ou à une pression de 0,1 à 4 bars, de préférence de 0,3 à 2 bars, plus préférentiellement de 0,5 à 1,5 bar.

Cette solution circule de préférence en contre-courant par rapport au flux gazeux à traiter et se charge ainsi en acide fluorohydrique. On récupère alors un flux gazeux comprenant de l'acide chlorhydrique et appauvri en HF, à l'issue de cette étape de lavage acide. Ce flux gazeux renferme par exemple moins de 500 ppm, voire moins de 100 ppm, de préférence au plus 50 ppm de HF.

Dans la seconde étape, dite d'adsorption adiabatique, du procédé selon l'invention, le flux gazeux d'acide chlorhydrique est absorbé dans de l'eau déminéralisée ou une solution aqueuse acide, pour former une solution aqueuse d'acide chlorhydrique, tandis que le composé fluoré d'intérêt est collecté sous forme gazeuse. La réaction d'absorption de l'HCl dans l'eau étant exothermique, il est préférable de limiter la pression à laquelle cette opération est conduite. En général, la pression est inférieure à 2 bars et de préférence inférieure à 1,5 bar. De cette façon, la température d'absorption n'excède pas 130°C, et de préférence 90°C. La concentration massique d'HCl dans la solution issue de l'étape d'adsorption adiabatique peut être de 5 à 50 %, de préférence de 15 à 40 %, et plus particulièrement de 30 à 35 % et la concentration en HF peut être par exemple inférieure à 200 ppm, de préférence d'au plus 100 ppm voire d'au plus 50 ppm.

La solution d'acide chlorhydrique issue de l'étape d'adsorption adiabatique est ensuite soumise à une étape d'adsorption d'impuretés sur lit de charbon actif. Cette étape peut être mise en œuvre dans des gammes de pression et de température qui ont déjà été indiquées ci-dessus en relation avec l'étape de lavage par une solution acide. On préfère que le lit de charbon actif soit préalablement lavé à l'acide chlorhydrique afin de limiter le relargage dans la solution de cendres ou de polluants minéraux ou métalliques. Cette étape permet de réduire le taux de composés organiques totaux d'environ 40 à 70%, par exemple de 50 à 60%, et le taux de composés organiques volatils de 60 à 80%, par exemple d'environ 70%. Les impuretés adsorbées par le lit de charbon actif sont en premier lieu des composés organiques volatils (COV).

A l'issue de l'étape d'adsorption d'impuretés, la température de la solution d'HCl doit être aussi faible que possible, et par exemple inférieure ou égale à 35°C, car l'étape suivante d'adsorption sur le gel de silice est exothermique. Au-dessus de cette température, l'efficacité d'adsorption diminue fortement. Le temps de contact de la solution d'acide chlorhydrique purifiée avec le gel de silice est compris entre quelques minutes et quelques heures (de préférence entre 10 et 60 min). Les vitesses de passage sont lentes et comprises entre 1 et 20 m/h et de préférence entre 3 et 10 m/h. La pression de fonctionnement est de quelques bars (de 1 à 7 bars et de préférence de 1 à 5 bars). Des exemples de gels de silice utilisables dans cette invention sont ceux commercialisés sous la référence Siogel® par CALDIC et sous la référence Fuji® B par FUJI SYLISIA CHEMICAL Ltd.

La teneur en fluorures de la solution d'HCl en entrée est de préférence inférieure ou égale à 100 ppm pour éviter tout risque de dégradation du gel de silice. Pour ce faire, une partie au moins de la solution traitée dans l'étape d'adsorption complémentaire, donc issue de l'étape (d), peut être recyclée dans cette étape, ce qui permet d'augmenter la concentration en acide chlorhydrique de la solution à traiter par le gel de silice et de réduire ainsi la concentration en HF dans cette solution, lorsque celle-ci est initialement supérieure à 100 ppm. Après cette étape d'adsorption complémentaire sur gel de silice, il est possible d'atteindre des teneurs en HF inférieures à 1 ppm dans la solution d'HCl.

Dans une forme d'exécution avantageuse, le procédé selon l'invention comprend une étape préliminaire de séparation des composés lourds, qui permet de condenser les goudrons. Cette étape consiste généralement à mettre le flux gazeux brut en contact avec une solution d'acide concentrée, sous pression, puis à désengager le gaz du liquide pour récupérer un flux gazeux dégoudronné.

En faisant à présent référence à la figure 1, l'installation 1 selon l'invention comprend quatre unités de traitement successives, à savoir une unité de lavage 3, une unité d'absorption adiabatique 4, une unité d'adsorption d'impuretés 5 et une unité d'adsorption complémentaire 6.

Dans la forme d'exécution illustrée à la Figure 1, le flux de produits réactionnels est amené d'abord vers une unité de séparation de composés lourds alimentée par une conduite de collecte 31 en sortie du réacteur catalytique. Cette unité vise à séparer le flux de produits réactionnels en un flux gazeux à traiter et en une fraction lourde contenant des goudrons. Cette unité de séparation est optionnelle. Elle comprend un éjecto-laveur 32 alimenté, d'une part, par le flux de produits réactionnels et, d'autre part, par une solution d'acide concentrée. L'éjecto-laveur assure la mise en contact des deux fluides et permet à la fois de relever le niveau de pression du gaz en utilisant la solution acide comme fluide moteur et d'abaisser la température du gaz. Le mélange de fluides est ensuite transféré vers un ballon flash 33 qui permet le désengagement du gaz par rapport au liquide. La fraction liquide est envoyée vers un décanteur 34 permettant de séparer les lourds. La solution acide surnageante est reprise par pompe et refroidie dans un échangeur avant d'être réinjectée dans l'éjecto-laveur. Une purge de la solution acide riche en HF est assurée en sortie de l'échangeur. Le flux gazeux issu du ballon flash est de son côté amené par une conduite d'alimentation 35 dans une colonne de lavage 36. La colonne de lavage 36 peut être une colonne à plateaux, telle qu'une colonne à plateaux perforés, ou à cloches, ou à clapets ou de type Duaiflow®. On préfère utiliser une colonne à plateaux à cloches. Pour limiter les phénomènes d'encrassement, on peut également utiliser une hauteur de garnissage vrac en pied de colonne. Le lavage du flux gazeux est effectué de préférence à contre-courant : le flux gazeux est alimenté en pied, et une solution d'acide est alimentée en tête, via une conduite d'amenée de solution acide 37. Au stade du lavage par la solution acide, la grande majorité de HF du flux gazeux passe en solution et est donc éliminée via une conduite de collecte de solution acide usée 38 en pied de colonne. Cette solution acide est refroidie dans un échangeur thermique avant d'être, pour partie, réintroduite dans la colonne de lavage 36 et, pour partie, réinjectée dans le décanteur 34 afin de compenser l'élimination de la solution acide en sortie du décanteur.

Le flux gazeux lavé issu de l'unité de lavage 3 est amené, via une conduite d'alimentation 41, dans l'unité d'adsorption adiabatique 4. L'unité d'absorption 4 permet d'absorber le HCl du flux gazeux dans une solution aqueuse, apportée par une conduite d'amenée de solution aqueuse 42. Cette solution aqueuse peut être simplement de l'eau déminéralisée, ou alternativement il peut s'agir d'une solution acide. Généralement, l'unité d'absorption 4 comporte une colonne 43 de mise en contact à contre-courant, la solution aqueuse étant fournie en tête et le flux gazeux en pied. Pour résister à la corrosion, la colonne 43 peut être en graphite ou bien en acier revêtu de polytétrafluoroéthylène (PTFE). Les internes de colonne peuvent être par exemple soit en graphite soit en polyfluorure de vinylidène (PVDF). Un flux gazeux désacidifié est récolté en tête, via une conduite de collecte de flux gazeux désacidifié 44. Ce flux gazeux renferme le composé fluoré d'intérêt, par exemple le fluorure de vinylidène.

Une solution d'HCl est récoltée en pied, via une conduite de collecte de solution d'acide chlorhydrique 45. Après refroidissement, cette solution est amenée, via une conduite d'amenée 46, vers une unité d'adsorption d'impuretés 5, qui comprend une ou plusieurs colonnes, ici deux colonnes 51 et 51', renfermant chacune un lit de charbon actif.

Après passage dans l'une et/ou l'autre de ces colonnes, la solution d'acide chlorhydrique purifiée est ensuite envoyée, via une conduite de collecte 63, vers une unité d'adsorption complémentaire 6, qui peut comprendre une ou plusieurs colonnes, ici deux colonnes 61 et 61', renfermant chacune un gel de silice. Il peut en effet être avantageux de disposer d'au moins deux colonnes 51, 51' et 61, 61', de façon à pouvoir changer de colonne une fois que la première est saturée, sans interrompre le procédé de purification d'acide chlorhydrique. L'installation 1 comprend en outre une conduite 62 de collecte de solution d'acide chlorhydrique purifiée issue de l'unité d'adsorption complémentaire 6.

La solution d'HCl purifiée récupérée à l'issue du procédé de l'invention peut être valorisée commercialement.

### EXEMPLES

Dans ces exemples, la concentration en ions fluorure (et donc en HF) est mesurée par ionométrie, selon la méthode des ajouts dosés, en utilisant une électrode au fluor.

Les composés organiques totaux (COT) sont déduits de la différence entre le carbone total et le carbone inorganiques, mesurés selon la norme NF-EN-1484.

Les composés organiques volatils (COV) sont mesurés en deux étapes : - une étape d'identification par chromatographie en phase gazeuse, couplée à un détecteur de masse en mode ionisation par électrons,
- une étape de quantification par chromatographie en phase gazeuse.

L'installation illustrée à la Figure 1 est montée en dérivation sur un atelier industriel de production de fluorure de vinylidène par pyrolyse catalytique de 1-chloro-1,1-difluoroéthane. Le flux de gaz brut issu du réacteur catalytique est constitué de : 33% en poids de fluorure de vinylidène (VF2), 19% en poids de HCl, 47% de 1-chloro-1,1-difluoroéthane et 600 ppm de HF.

Ce flux gazeux brut est envoyé dans l'éjecto-laveur 32 de l'unité de séparation de composés lourds, à un débit de 5120 kg/h. La température dans l'éjecto-laveur est de 70°C et la pression est réglée à 2,4 bars. Le flux gazeux issu de cette unité de séparation est débarrassé des composés lourds susceptibles de former des goudrons. Il est envoyé au pied d'une colonne à plateaux 36, à un débit de 5120 kg/h. La colonne a une hauteur de 1,20 mètres et cinq centimètres de diamètre. Elle comporte vingt plateaux perforés (200 perforations par plateau, chacune faisant 1,75 mm de diamètre). L'écartement entre les plateaux est égal au diamètre de la colonne. Chaque plateau comporte un déversoir permettant la circulation du liquide vers le pied de colonne tandis que les perforations autorisent le passage du gaz vers la tête de colonne. Le matériau préféré pour la colonne est le PVDF. La colonne est alimentée par une solution d'acide chlorhydrique liquide commerciale à 33% en poids, renfermant moins de 5 ppm de HF.

Cette solution est introduite dans la colonne 36 à un débit de 285 kg/h, à une température de 35°C et sous une pression de 1,4 bar. La température de la colonne est de 30°C et la pression est maintenue à 1,7 bar. Le liquide en pied de colonne renferme 59% en poids d'eau, 40% en poids d'HCl et 0,85% en poids de HF. Sa température est abaissée à 30°C avant de le réinjecter, pour partie, dans la colonne et d'envoyer l'autre partie vers le décanteur 34. Le gaz sortant en tête de la colonne de lavage 36 à un débit de 5080 kg/h comprend 33,3% en poids de VF2, 47,3% en poids de 1-chloro-1,1-difluoroéthane, 18,5% de HCl et 100 ppm de HF.

Le flux gazeux issu de la colonne 36 est traité ensuite sur la colonne adiabatique 43, qui est alimentée en eau déminéralisée, permettant l'absorption de l'acide chlorhydrique. Le gaz sortant en tête de colonne adiabatique renferme essentiellement du VF2 et du CClF₂-CH₃ (50/50 en % molaire) et ne contient que des traces d'HCl. On obtient en pied de colonne adiabatique une solution d'HCl concentrée à 33%, contenant des composés organo-halogénés résultant de la pyrolyse, majoritairement des composés chlorés en C₂ tels que Cl₂C=CCl₂ (F1110), CHCl₂-CHCl₂ (F130), CH₂Cl-CCl₃ (F130A), CH₂Cl-CHF₂ (F142), ainsi que du CClF₂-CH₃ (F142B) résiduel. Cette solution renferme 200 ppm de composés organiques totaux (COT) et 100 ppm de composés organiques volatils (COV).

Ces composés organiques sont ensuite séparés par passage de cette solution aqueuse sur le lit de charbon actif 51 de l'unité d'adsorption d'impuretés 5, à un débit de 2850 kg/h. La température du lit est de 35°C et la pression est de 0,4 bar. Cette étape permet de réduire les taux de COT et COV à 100 ppm et 30 ppm, respectivement.

La solution aqueuse sortant de l'unité 5 est dirigée vers l'unité d'adsorption complémentaire 6, dans la colonne 61, à un débit de 7600 kg/h. La colonne 61 comprend un lit de gel de silice maintenu à une température de 35°C, qui se sature en HF au contact de la solution. La solution aqueuse de HCl récupérée en pied de colonne est pour partie dirigée vers une cuve de stockage, l'autre partie étant recyclée en tête de la colonne 61 et mélangée à la solution aqueuse provenant de l'unité d'adsorption d'impuretés. Il est ainsi possible de réduire la concentration en HF de 200 ppm à 75 ppm en entrée de colonne et de favoriser le traitement complémentaire sur gel de silice. Le débit de recyclage est dans cet exemple de 4750 kg/h. On obtient ainsi une solution aqueuse d'acide chlorhydrique renfermant une quantité indétectable de HF. Le lit est ensuite régénéré par lavage à l'eau. Une solution contenant de l'acide fluorhydrique dilué est ainsi générée, qui doit être détruite.

## Revendications

1. Procédé de traitement d'un flux gazeux contenant de l'acide chlorhydrique, de l'acide fluorhydrique, un composé fluoré d'intérêt et des composés organiques halogénés, dans lequel le flux gazeux est successivement soumis à :
(a) une étape de lavage par une solution acide, telle qu'une solution d'acide chlorhydrique, pour obtenir un flux gazeux lavé ;
(b) une étape d'absorption adiabatique dans une solution aqueuse de l'acide chlorhydrique contenu dans ledit flux gazeux lavé, permettant de collecter une solution d'acide chlorhydrique et un flux gazeux renfermant ledit composé fluoré d'intérêt ;
(c) une étape d'adsorption sur charbon actif des impuretés organiques présentes dans ladite solution d'acide chlorhydrique, pour obtenir une solution d'acide chlorhydrique purifiée ; et
(d) une étape de mise en contact de ladite solution d'acide chlorhydrique purifiée avec un gel de silice.

2. Procédé selon la revendication 1, **caractérisé en ce que** ledit flux gazeux mis en œuvre dans l'étape (a) est un flux gazeux brut issu d'une réaction de synthèse du composé fluoré d'intérêt, tel que le fluorure de vinylidène.

3. Procédé selon la revendication 1, **caractérisé en ce que** ledit flux gazeux mis en œuvre dans l'étape (a) résulte de la séparation des composés lourds contenus dans le flux gazeux brut issu d'une réaction de synthèse d'un composé fluoré d'intérêt, tel que le fluorure de vinylidène.

4. Procédé selon la revendication 3, **caractérisé en ce qu'**il comprend une étape préliminaire de séparation des composés lourds consistant à mettre le flux gazeux brut en contact avec une solution d'acide concentrée, sous pression, puis à désengager le gaz du liquide pour récupérer un flux gazeux dégoudronné.

5. Procédé selon la revendication 2 ou 3, **caractérisé en ce que** la synthèse du composé fluoré d'intérêt est effectuée par pyrolyse, catalytique ou non, d'un composé organofluoré comprenant au moins un atome de chlore, ou par fluoration catalytique d'un composé organochloré avec de l'acide fluorhydrique, de préférence par pyrolyse catalytique d'un composé organofluoré comprenant au moins un atome de chlore.

6. Procédé selon l'une quelconque des revendications 1 à 5, **caractérisé en ce qu'**une partie au moins de la solution issue de l'étape (d) est recyclée dans l'étape (d).

7. Procédé selon l'une quelconque des revendications 1 à 6, **caractérisé en ce que** la solution aqueuse d'acide chlorhydrique issue de l'étape (d) renferme au moins 30% en poids d'acide chlorhydrique, moins de 10 ppm, voire moins de 5 ppm, mieux, moins de 1 ppm, d'acide fluorhydrique et moins de 150 ppm, voire moins de 100 ppm, mieux, moins de 50 ppm, de composés organiques halogénés.

8. Procédé selon l'une quelconque des revendications 1 à 7, **caractérisé en ce que** le flux gazeux n'est pas préalablement soumis à une étape de distillation.

9. Installation (1) de traitement d'un flux gazeux brut contenant de l'acide chlorhydrique, de l'acide fluorhydrique, un composé fluoré d'intérêt et des composés organiques halogénés, comprenant :
- une unité de lavage (3) comprenant une colonne de lavage (36) alimentée, d'une part, par une conduite d'amenée (35) dudit flux gazeux brut et, d'autre part, par une conduite d'amenée (37) de solution acide ;
- une unité d'absorption adiabatique (4) comprenant une colonne (43) alimentée, d'une part, par une conduite de collecte (41) de flux lavé, issue de l'unité de lavage (3), et, d'autre part, par une conduite d'amenée (42) de solution aqueuse ;
- une conduite de collecte (44) du composé fluoré d'intérêt sous forme gazeuse, en sortie de colonne (43) de l'unité d'adsorption adiabatique (4),
- une unité d'adsorption d'impuretés organiques (5), comprenant un lit de charbon actif alimenté par une conduite de collecte (46) de solution d'acide chlorhydrique issue de l'unité d'adsorption adiabatique (4) ;
- une unité d'adsorption complémentaire (6) reliée à une conduite de collecte (63) de solution purifiée issue de l'unité d'adsorption d'impuretés (5), et comprenant une colonne (61) renfermant un gel de silice.

10. Installation selon la revendication 9, **caractérisée en ce qu'**elle comprend en outre, au sein de l'unité de lavage (3), une unité de séparation de composés lourds disposée en amont de la colonne de lavage, ladite unité de séparation de composés lourds étant alimentée par une conduite d'amenée (31) du flux gazeux brut et reliée à la colonne de lavage (36) par une conduite de collecte (35) de flux gazeux dégoudronné.

11. Procédé de préparation d'un composé fluoré d'intérêt comprenant :
- la pyrolyse, catalytique ou non, d'un composé organofluoré comprenant au moins un atome de chlore ou la fluoration catalytique d'un composé organochloré avec de l'acide fluorhydrique,
- la collecte d'un flux gazeux brut contenant de l'acide chlorhydrique, de l'acide fluorhydrique, ledit composé fluoré d'intérêt et des composés organiques halogénés ; et
- le traitement dudit flux gazeux brut suivant le procédé selon l'une quelconque des revendications 1 à 8.

## Patentansprüche

1. Verfahren zur Behandlung eines Gasstroms, der Chlorwasserstoffsäure, Fluorwasserstoffsäure, eine Fluorverbindung von Interesse und organische Halogenverbindungen enthält, wobei der Gasstrom nacheinander Folgendem unterworfen wird:
(a) einem Schritt des Waschens mit einer Säurelösung, wie einer Chlorwasserstoffsäurelösung, zum Erhalt eines gewaschenen Gasstroms;
(b) einen Schritt der adiabatischen Absorption der in dem gewaschenen Gasstrom enthaltenen Chlorwasserstoffsäure in einer wässrigen Lösung, was das Sammeln einer Chlorwasserstoffsäurelösung und eines Gasstroms, der die Fluorverbindung von Interesse enthält, ermöglicht;
(c) einen Schritt der Adsorption der in der Chlorwasserstoffsäurelösung vorliegenden organischen Verunreinigungen an Aktivkohle zum Erhalt einer gereinigten Chlorwasserstoffsäurelösung; und
(d) einen Schritt des Inkontaktbringens der gereinigten Chlorwasserstoffsäurelösung mit einem Kieselgel.

2. Verfahren nach Anspruch 1, **dadurch gekennzeichnet, dass** es sich bei dem in Schritt (a) eingesetzten Gasstrom um einen rohen Gasstrom handelt, der aus einer Umsetzung zur Synthese der Fluorverbindung von Interesse, wie Vinylidenfluorid, stammt.

3. Verfahren nach Anspruch 1, **dadurch gekennzeichnet, dass** der in Schritt (a) eingesetzte Gasstrom aus der Abtrennung der in dem Gasstrom, der aus einer Umsetzung zur Synthese einer Fluorverbindung von Interesse, wie Vinylidenfluorid, stammt, enthaltenen schweren Verbindungen resultiert.

4. Verfahren nach Anspruch 3, **dadurch gekennzeichnet, dass** es einen vorgeschalteten Schritt der Abtrennung der schweren Verbindungen umfasst, der darin besteht, dass man den rohen Gasstrom unter Druck mit einer konzentrierten Säurelösung in Kontakt bringt und dann das Gas von der Flüssigkeit ablässt, wobei man einen entteerten Gasstrom gewinnt.

5. Verfahren nach Anspruch 2 oder 3, **dadurch gekennzeichnet, dass** die Synthese der Fluorverbindung von Interesse durch katalytische oder nichtkatalytische Pyrolyse einer Organofluorverbindung mit mindestens einem Chloratom oder durch katalytische Fluorierung einer Organochlorverbindung mit Fluorwasserstoffsäure, vorzugsweise durch katalytische Pyrolyse einer Organofluorverbindung mit mindestens einem Chloratom, durchgeführt wird.

6. Verfahren nach einem der Ansprüche 1 bis 5, **dadurch gekennzeichnet, dass** mindestens ein Teil der Lösung aus Schritt (d) in Schritt (d) zurückgeführt wird.

7. Verfahren nach einem der Ansprüche 1 bis 6, **dadurch gekennzeichnet, dass** die wässrige Chlorwasserstoffsäurelösung aus Schritt (d) mindestens 30 Gew.-% Chlorwasserstoffsäure, weniger als 10 ppm, sogar weniger als 5 ppm, noch besser weniger als 1 ppm, Fluorwasserstoffsäure und weniger als 150 ppm, sogar weniger als 100 ppm, noch besser weniger als 50 ppm, organische Halogenverbindungen enthält.

8. Verfahren nach einem der Ansprüche 1 bis 7, **dadurch gekennzeichnet, dass** der Gasstrom nicht vorher einem Destillationsschritt unterworfen wird.

9. Anlage (1) zur Behandlung eines rohen Gasstroms, der Chlorwasserstoffsäure, Fluorwasserstoffsäure, eine Fluorverbindung von Interesse und organische Halogenverbindungen enthält, die Folgendes umfasst:
- eine Wascheinheit (3) mit einer Waschsäule (36), die einerseits durch eine Leitung (35) zur Zuführung des rohen Gasstroms und andererseits durch eine Leitung (37) zur Zuführung von Säurelösung gespeist wird;
- eine Einheit (4) zur adiabatischen Absorption mit einer Säule (43), die einerseits durch eine Leitung (41) zum Sammeln des gewaschenen Stroms aus der Wascheinheit (3) und andererseits durch eine Leitung (42) zur Zuführung von wässriger Lösung gespeist wird;
- eine Leitung (44) zum Sammeln der Fluorverbindung von Interesse in gasförmiger Form am Ausgang der Säule (43) der Einheit (4) zur adiabatischen Adsorption;
- eine Einheit (5) zur Adsorption von organischen Verunreinigungen mit einer Aktivkohleschüttung, die durch eine Leitung (46) zum Sammeln von Chlorwasserstoffsäurelösung aus der Einheit (4) zur adiabatischen Adsorption gespeist wird;
- eine zusätzliche Adsorptionseinheit (6), die mit einer Leitung (63) zum Sammeln von gereinigter Lösung aus der Einheit (5) zur Adsorption von Verunreinigungen verbunden ist und eine ein Kieselgel enthaltende Säule (61) umfasst.

10. Anlage nach Anspruch 9, **dadurch gekennzeichnet, dass** sie außerdem in der Wascheinheit (3) eine Einheit zur Abtrennung von schweren Verbindungen umfasst, die stromaufwärts der Waschsäule angeordnet ist, wobei die Einheit zur Abtrennung von schweren Verbindungen durch eine Leitung (31) zur Zuführung des rohen Gasstroms gespeist wird und über eine Leitung (35) zum Sammeln von entteertem Gasstrom mit der Waschsäule (36) verbunden ist.

11. Verfahren zur Herstellung einer Fluorverbindung von Interesse, das Folgendes umfasst:
- katalytische oder nichtkatalytische Pyrolyse einer Organofluorverbindung mit mindestens einem Chloratom oder katalytische Fluorierung einer Organochlorverbindung mit Fluorwasserstoffsäure,
- Sammeln eines rohen Gasstroms, der Chlorwasserstoffsäure, Fluorwasserstoffsäure, die Fluorverbindung von Interesse und organische Halogenverbindungen enthält; und
- Behandlung des rohen Gasstroms gemäß dem Verfahren nach einem der Ansprüche 1 bis 8.

## Claims

1. A process for the treatment of a gas stream containing hydrochloric acid, hydrofluoric acid, a fluorinated compound of interest and halogenated organic compounds, in which the gas stream is successively subjected to:
(a) a stage of washing with an acid solution, such as a hydrochloric acid solution, in order to obtain a washed gas stream;
(b) a stage of adiabatic absorption in an aqueous solution of the hydrochloric acid present in said washed gas stream, making it possible to collect a hydrochloric acid solution and a gas stream including said fluorinated compound of interest;
(c) a stage of adsorption on active carbon of the organic impurities present in said hydrochloric acid solution, in order to obtain a purified hydrochloric acid solution; and
(d) a stage of bringing said purified hydrochloric acid solution into contact with a silica gel.

2. The process as claimed in claim 1, **characterized in that** said gas stream employed in stage (a) is a crude gas stream resulting from a reaction for the synthesis of the fluorinated compound of interest, such as vinylidene fluoride.

3. The process as claimed in claim 1, **characterized in that** said gas stream employed in stage (a) results from the separation of the heavy compounds present in the crude gas stream resulting from a reaction for the synthesis of a fluorinated compound of interest, such as vinylidene fluoride.

4. The process as claimed in claim 3, **characterized in that** it comprises a preliminary stage of separation of the heavy compounds which consists in bringing the crude gas stream into contact with a concentrated acid solution, under pressure, and in then releasing the gas from the liquid in order to recover a detarred gas stream.

5. The process as claimed in claim 2 or 3, **characterized in that** the synthesis of the fluorinated compound of interest is carried out by catalytic or noncatalytic pyrolysis of an organofluorine compound comprising at least one chlorine atom or by catalytic fluorination of an organochlorine compound with hydrofluoric acid, preferably by catalytic pyrolysis of an organofluorine compound comprising at least one chlorine atom.

6. The process as claimed in any one of claims 1 to 5, **characterized in that** a portion at least of the solution resulting from stage (d) is recycled in stage (d).

7. The process as claimed in any one of claims 1 to 6, **characterized in that** the aqueous hydrochloric acid solution resulting from stage (d) includes at least 30% by weight of hydrochloric acid, less than 10 ppm, indeed even less than 5 ppm, better still less than 1 ppm, of hydrofluoric acid and less than 150 ppm, indeed even less than 100 ppm, better still less than 50 ppm, of halogenated organic compounds.

8. The process as claimed in any one of claims 1 to 7, **characterized in that** the gas stream is not subjected beforehand to a distillation stage.

9. A plant (1) for the treatment of a crude gas stream containing hydrochloric acid, hydrofluoric acid, a fluorinated compound of interest and halogenated organic compounds, comprising:
- a washing unit (3) comprising a washing column (36) fed, on the one hand, by a pipe (35) for conveying said crude gas stream and, on the other hand, by a pipe (37) for conveying acid solution;
- an adiabatic absorption unit (4) comprising a column (43) fed, on the one hand, by a pipe (41) for collecting washed stream resulting from the washing unit (3) and, on the other hand, by a pipe (42) for conveying aqueous solution;
- a pipe (44) for collecting the fluorinated compound of interest in gaseous form, at the outlet of the column (43) of the adiabatic adsorption unit (4);
- a unit for adsorption of organic impurities (5), comprising an active carbon bed fed by a pipe (46) for collecting hydrochloric acid solution resulting from the adiabatic adsorption unit (4);
- an additional adsorption unit (6) connected to a pipe (63) for collecting purified solution resulting from the unit for adsorption of impurities (5), and comprising a column (61) including a silica gel.

10. The installation as claimed in claim 9, **characterized in that** it additionally comprises, within the washing unit (3), a unit for separation of heavy compounds, which is positioned upstream of the washing column, said unit for separation of heavy compounds being fed by a pipe (31) for conveying the crude gas stream and connected to the washing column (36) by a pipe (35) for collecting detarred gas stream.

11. A process for the preparation of a fluorinated compound of interest, comprising:
- the catalytic or noncatalytic pyrolysis of an organofluorine compound comprising at least one chlorine atom or the catalytic fluorination of an organochlorine compound with hydrofluoric acid:
- the collection of a crude gas stream containing hydrochloric acid, hydrofluoric acid, said fluorinated compound of interest and halogenated organic compounds; and
- the treatment of said crude gas stream according to the process as claimed in any one of claims 1 to 8.
